# EUROPEAN PATENT APPLICATION

(11) **EP 2 543 369 A2**
(43) Date of publication of application: **09.01.2013**
(21) Application number: 11719586.7
(22) Date of filing: 04.03.2011
(51) Int. Cl.: A61K 31/165, A61K 33/06, A61K 33/14, A61P 7/08, A61P 13/12, A61P 17/18

(54) **SOLUTION FOR PERITONEAL DIALYSIS**

(30) Priority: 05.03.2010 ES 201030326
(71) Applicant: Universidad De Castilla La Mancha, 02071 Albacete (ES)
(72) Inventor: CEÑA CALLEJO, Valentín, E-02071 Albacete (ES); PÉREZ MARTÍNEZ, Francisco Carlos, E-02071 Albacete (ES); PÉREZ MARTÍNEZ, Juan, E-02071 Albacete (ES); GÓMEZ ROLDÁN, Carmen, E-02071 Albacete (ES); ORTEGA CERRATO, Agustín, E-02071 Albacete (ES); LLAMAS FUENTES, Francisco, E-02071 Albacete (ES)
(74) Representative: Pons Ariño, Angel
(86) International application number: PCT/ES2011/070142
(87) International publication number: WO 2011/107649

(57) **Abstract**

The invention relates to a type of solution for peritoneal dialysis, comprising Aliskiren or one of the derivatives thereof, at least one electrolyte, at least one buffer solution, and at least one osmotic agent. The invention reduces the toxicity of commonly used peritoneal dialysis solutions on mesothelial peritoneal cells. The invention also relates to the use of this type of solution for processes requiring peritoneal dialysis, such as in the case of chronic renal failure

## Description

The present invention relates to a type of peritoneal dialysis solution comprising aliskiren, at least one electrolyte, at least one buffer solution and at least one osmotic agent. This will allow a reduction in toxicity of these habitual peritoneal dialysis solutions with respect to peritoneal mesothelial cells. On the other hand, the present invention relates to the use of this type of solutions for processes wherein peritoneal dialysis is required, as in the case of chronic renal failure.

### PRIOR ART

Peritoneal dialysis (PD) is an efficient therapy for chronic renal failure, wherein the peritoneal membrane is used as a semi-permeable membrane for removing harmful substances such as urea and potassium from the blood, as well as excess liquid. Patients subjected to this treatment have the advantage of being able to lead an active life without frequent hospital stays, due to the fact that PD is normally carried out at the patient's home or workplace (Grassmann A, Gioberge S, Moeller S, et al. Nephrol Dial Transplant 2005; 20: 2587-93 *and* Burkart JM, Nolph KD. NIDDK 2006; 6: 1-24*).*

The main drawback of this dialysis method is the tendency towards peritoneal infection (peritonitis), although its incidence has decreased dramatically since the appearance of new connection systems specially designed to ensure maximum sterility. On the other hand, the continuous exposure to dialysis liquid entails deterioration in the dialysis capacity of the peritoneal membrane, which is associated to fibrosis and vasculopathies, although the factors that influence the speed of deterioration of the peritoneal function are not totally clear (Gillerot G, Devuyst O. Clin Nephrol 2003; 60: 1-6)*.*

Alteration of the mesothelial cells that form the peritoneal membrane as a result of continuous exposure to dialysis liquid with high osmolarity and a concentration of glucose can be one of the mechanisms involved in peritoneal dysfunction (Coles GA, Topley N. Adv Ren Replace Ther 2000; 7: 289-301)*.* Many types of stimuli are known to induce apoptosis, such as cytokines, hormones, infections or drugs (Catalan MP, Subirá D, Reyero A, et al. Kidney Int 2003; 64: 321-30; Zheng Z, Ye R, Yu X, et al. Adv Perit Dial 2001; 17: 53-7)*.* Additionally, high concentrations of glucose are known to induce apoptosis in many cell types, including mesothelial cells (Sharifi AM, Mousavi SH, Farhadi M, et al. J Pharmacol Sci 2007; 104: 258-62*).*

In addition to the studies carried out on peritoneal mesothelial cell cultures, the most frequently used PD animal model is the rat (González-Mateo GT, Loureiro-Alvarez J, Rayego-Mateos S, et al. Nefrologia 2008; 28 (Suppl 6): 17-22). Earlier studies carried out on these models have focused on the biocompatibility of dialysis liquids, the inflammatory response developed in the peritoneum against exposure to dialysis liquids, angiogenesis and the importance of the vascular network in the permeability of the peritoneal membrane or fibrosis, and the contribution of peritonitis to peritoneal membrane damage (Krediet RT, Zweers MM, Van der Wal AC, et al. Perit Dial Int 2000; 20 (Suppl 2): S19-25*;* Bazargani F. Swed Dent J Suppl 2005; 171: 1-57*;* Hurst SM, McLoughlin RM, Monslow J, et al. J Immunol 2002; 169: 5244-51*;* Kim YL, Do J, Park SH, et al. Nephrology (Carlton) 2003; 8 (Suppl): S28-32*;* Gillerot G, Devuyst O. Clin Nephrol 2003; 60: 1-6*;* Lameire N, Van Biesen W, Mortier S, et al. Contrib Nephrol 2006; 150: 70-6*;* Margetts PJ, Bonniaud P, Liu L, et al. J Am Soc Nephrol 2005; 16: 425-36*;* Margetts PJ, Kolb M, Galt T, et al. J Am Soc Nephrol 2001; 12: 2029-39)*.*

PD research dates back to the mid-twentieth century, when it was first studied in cases of uremia and, subsequently, in cases of acute or chronic renal failure. However, PD as it is currently understood in its continuous outpatient modality, was conceived in 1978 by Popovich RP, Moncrief JW, Nolph KD, et al. J Am Soc Nephrol 1999; 10: 901-10*.* Since then, its use has become increasingly widespread, extending to more than 180,000 patients worldwide *(*Lysaght MJ. J Am Soc Nephrol 2002; 13 (Suppl 1): S37-40*).* Growth in recent years has been very diverse in each different country, reaching 90% in Mexico, 30-50% in Scandinavian countries and Canada, 17% in the USA, 8-11% in Central and South European countries and 5% in Japan (Lysaght MJ. J Am Soc Nephrol 2002; 13 (Suppl 1): S37-40)*.*

Every year, one-third of PD patients are transferred to haemodialysis after loss of peritoneal membrane effectiveness (Kawaguchi Y. Perit Dial Int 1999; 19: S327-8)*,* due to which research must continue in order to achieve an increase in the dialytic potential of the peritoneal membrane and extend its average lifespan in chronic renal failure patients. This will probably be achieved using pharmacological additives in the dialysis liquid such as that presented in the present invention.

The approximate cost of dialysis per year and per patient amounts to approximately €60,000. Taking into account the high number of patients who currently require dialysis treatment, the global cost of treating renal failure totals approximately €60,000 million per year. The world's population is growing at a rate of 1.3%, while the growth rate of the population on dialysis is growing at an annual rate of 7-8% (Lysaght MJ. J Am Soc Nephrol 2002; 13 (Supl 1): S37-40)*.* We are obviously undergoing enormous worldwide growth in the dialysis population, in such a manner that by 2010 there will be approximately two million patients, with the ensuing economic costs.

Of those renal failure patients on substitutive treatment, 68% are being treated with haemodialysis, 23% live with a kidney transplant and only 9% with PD (Lysaght MJ. J Am Soc Nephrol 2002; 13 (Supl 1): S37-40)*.* The evolution of patients treated with PD has improved significantly, due to which it is currently accepted as a treatment equivalent to haemodialysis. In recent years, PD has proven to be as effective as haemodialysis, at least in the first four or five years of use, in terms of life expectancy and quality, and with a good cost/effectiveness ratio.

To date, PD has proven its usefulness and has become established as an excellent alternative to haemodialysis, even as an initial substitutive treatment due to its positive qualities established beyond all doubt:
- Less expensive than haemodialysis, particularly in the Western world.
- Life expectancy similar to that of haemodialysis and higher during the first two to three years.
- Preferred treatment for children and young people.
- Optimum treatment prior to kidney transplant.

However, in order for PD to achieve widespread acceptance and increase its use percentage, different problems must be solved which detracts from its greater use. Two important issues are:
- PD has a higher failure rate compared to haemodialysis. In fact, research must continue in order to achieve a lower medium and long-term ultrafiltration failure rate, improved solute clearance and lower incidence of peritonitis.
- It is still impossible to maintain a patient on prolonged PD (more than ten years) due to the alterations caused in the long term to the structure and function of the peritoneal membrane. In this regard, the research carried out on new pharmacological treatments that will avoid this damage is crucial to increasing the use of PD and reducing the cost currently entailed by the use of haemodialysis.

The low pH of the dialysis liquid, its high osmotic power, the mechanical process of introducing and withdrawing the liquid, glucose and its degradation products render peritoneal dialysis a bio-incompatible process capable of damaging the peritoneal cavity. For this reason, further study at molecular and cellular level is required to assess the in vitro and in vivo effects of exposure to these solutions, as well as the study of new pharmacological treatments that will minimize the adverse side effects that could appear in patients that require PD.

Apoptosis or programmed cell death is a genetically controlled cellular process whereby cells induce their own death in response to certain stimuli. Exposure to PD liquids increases the generation of reactive oxygen species (ROS) and induces apoptosis in peritoneal mesothelial cells (Yang AH, Chen JY, Lin YP, et al. Kidney Int 1997; 51: 1280-8)*.* These two processes can be related, as ROS is known to activate p38 MAPKs and trigger an apoptotic process, increasing the expression of proinflammatory mediators that could intensify the cytotoxicity of dialysis liquids. It has been observed that, during induced apoptosis, an increase in phosphorylation of p38 MAPK occurs (Sepúlveda JC, Moreno Manzano V, Alique M, et al. Nefrología 2005; 25: 131-40) and that, on the contrary, anti-apoptotic treatments decrease the phosphorylation of p38 MAPK (Vorobiov M, Malki M, Shnaider A, et al. Perit Dial Int 2008; 28: 648-54). For this reason, the measurement of phosphoprotein 38 (phospho-p38) MAPK serves to evaluate the anti-apoptotic effect of different agents.

Another process triggered by exposure to peritoneal dialysis liquids is the production of fibronectin (Ha H, Yu MR, Lee HB. Kidney Int 2001; 59: 463-70*;* Lee HB, Yu MR, Song JS, et al. Kidney Int 2004; 65: 1170-9). This protein is important in fibrosing processes and in the epithelial-mesenchymal transition of peritoneal mesothelial cells. Both processes are related to the loss of peritoneal dialytic capacity.

In patients treated with PD, therapeutic complements that will lessen the damage caused by dialysis liquids in the long term are being sought.

Renin is the first enzyme of the renin-angiotensin-aldosterone axis, which plays a vital role in the control of arterial pressure. Renin transforms angiotensinogen into angiotensin I which, by means of the angiotensin-converting enzyme (ACE), is converted into angiotensin II. On one hand, it originates artery smooth muscle contraction, leading to vasoconstriction and increased arterial pressure. On the other hand, it increases aldosterone synthesis, which originates retention of sodium and water in the renal tubules, increased plasma volume and, indirectly, increased arterial pressure. Aliskiren (W02007147596 (A1)) binds to the S3^{bp} protein, which is necessary for the conversion of angiotensin I to angiotensin II, whereupon renin synthesis is impeded (Rahuel J, Rasetti V, Maibaum J, et al. Chem Biol 2000;7:493-504)*.*

Therefore, new solutions for peritoneal dialysis that will solve all of the aforementioned problems must be developed. In this manner, a new type of dialysis solution that prevents the deficiencies of the other solutions disclosed in the state of the art has been developed in the present invention.

### DESCRIPTION OF THE INVENTION

The present invention relates to peritoneal dialysis solutions that comprise aliskiren, at least one electrolyte, at least one buffer solution and at least one osmotic agent for the treatment of renal failure patients subjected to peritoneal dialysis.

This enables dual functionality, the first or classic function belonging to the dialysis liquids and the second due to aliskiren, which reduces the toxicity of these solutions with respect to peritoneal mesothelial cells. This is due to the reduction in reactive oxygen species, the phosphorylation of p38 MAPK and the activation of caspase-3, processes that can be generated by the high glucose concentrations contained in peritoneal dialysis solutions.

Consequently, the peritoneal dialysis solution of the present invention prevents oxidative stress, apoptotic processes and ensuing peritoneal damage, consequently prolonging the time during which the patient can use peritoneal dialysis before being transferred to haemodialysis.

Therefore, a first essential aspect of the present invention relates to a peritoneal dialysis solution comprising the following elements:
a) at least one electrolyte;
b) at least one buffer solution;
c) at least one osmotic pressure regulating agent;
d) aliskiren or any of its derivatives.

In a preferred embodiment, the electrolyte(s) are selected from the group formed by the following electrolytes: Na⁺, K⁺, Mg²⁺, Ca²⁺, Cl⁻ of different salts or any combination thereof. The concentration of electrolyte to be added is: Na 100-200 mmol/l; K 0-4 mmol/l; Mg 0.1-2 mmol/l; Ca 0.5-3 mmol/l; Cl 50-200 mmol/l.

In another preferred embodiment, the osmotic agent of the peritoneal dialysis solution used to remove the excess water from the blood is selected in a non-limiting manner from the group formed by glucose, polyglucose, mannitol, glycerol, amino acids, polypeptides or any combination thereof. The amount of osmotic agent to be added ranges from 100 to 600 mOsm/kg.

According to another preferred embodiment, aliskiren is used in the peritoneal dialysis solution at a concentration of between 50nM and 1mM, due to its inhibiting effects on the mechanisms responsible for the toxicity of the peritoneal dialysis liquids with the respect to the peritoneum including, among others, the production of oxygen-free radicals due to its inhibiting effects on the p38 MAPK channel and caspase-3 channel.

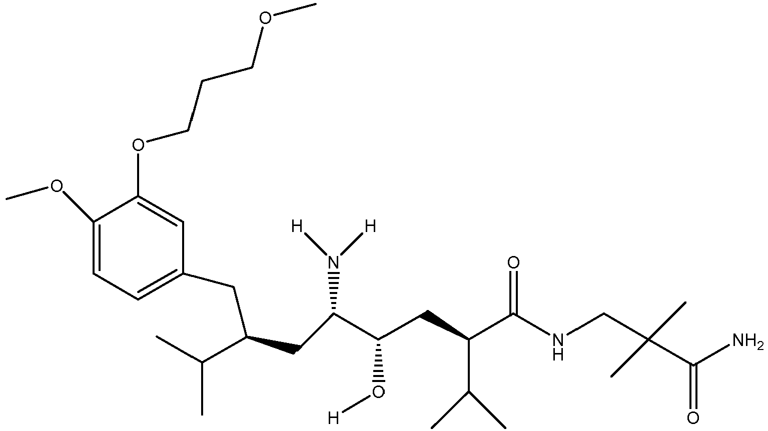

A second essential aspect of the present invention relates to the use of the peritoneal dialysis solution for the preparation of a medicament.

A preferred embodiment relates to the use of the dialysis solution for the preparation of a medicament for preventing and/or treating renal diseases.

Another preferred embodiment relates to the use of the dialysis solution for the preparation of a medicament for preventing and/or treating toxicity with respect to peritoneal mesothelial cells.

Another preferred embodiment relates to the use of the dialysis solution for the preparation of a medicament for preventing and/or treating reactive oxygen species, phosphorylation of the p38 MAPK protein and activation of caspase-3.

Another preferred embodiment relates to the use of the dialysis solution for the preparation of a medicament for preventing and/or treating oxidative stress, apoptotic processes, gene expression entailing damage to peritoneal cells and peritoneal damage generated by the dialysis liquids.

Another preferred embodiment relates to the use of the dialysis solution for the preparation of a medicament for preventing and/or treating chronic renal failure.

Normally, the peritoneal dialysis solution of the present invention is administered four times a day, in a volume of 2 litres per dose. The solution is administered to patients suffering from chronic renal failure in accordance to the methods normally used for performing peritoneal dialysis. More specifically, the solution is administered to the peritoneum via a catheter pre-implanted in the peritoneum. Normally, around 4-6 hours are required to remove the metabolic waste accumulated in the blood of patients with renal failure.

Examples of the methodology and results that support the present invention are described below, although the scope of the present invention is not limited thereto.

### BRIEF DESCRIPTION OF THE FIGURES

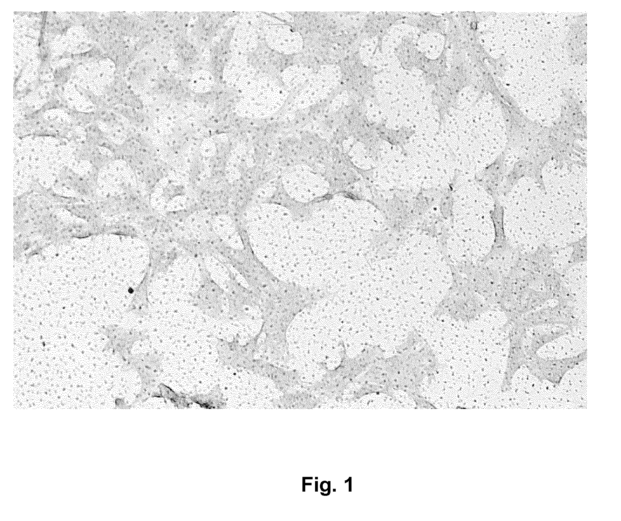

Figure 1 shows an immunohistochemical staining with positive HBME-1 in primary culture of rat peritoneal mesothelial cells (100x).

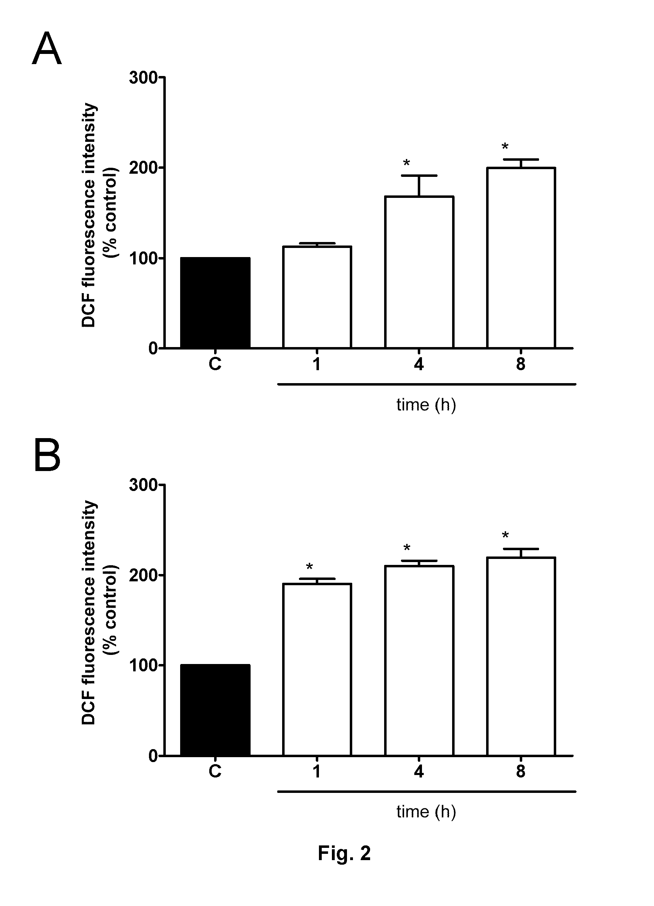

Figure 2 shows the effects of D-glucose at 50 mM (A) and 83 mM (B) on the production of reactive oxygen species (ROS) in rat PMCs. After incubating the cells in a carrier or D-glucose (50 mM or 83 mM) for 1-8 hours, dichlorofluorescein (DCF) intensity was measured using flow cytometry, as described in Material and Methods. The data is expressed as mean ± SEM, n=3. * p <0.05 compared to the carrier (V).

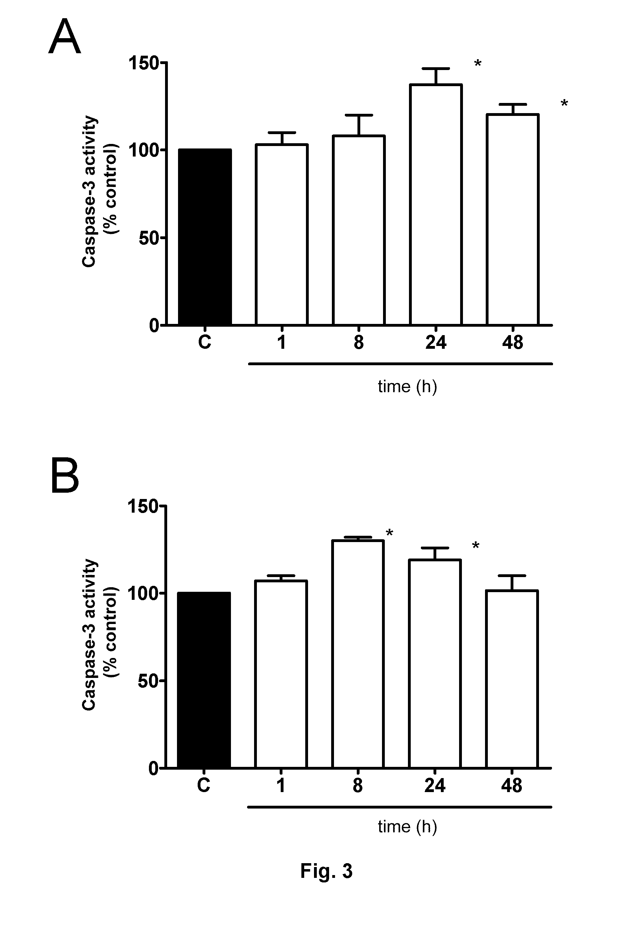

Figure 3 shows the role of caspase-3 in cell toxicity mediated by 50 mM (A) and 83 mM (B) of D-glucose in PMCs. The data is expressed as mean ± SEM, n=3. * p <0.05 compared to the carrier (V).

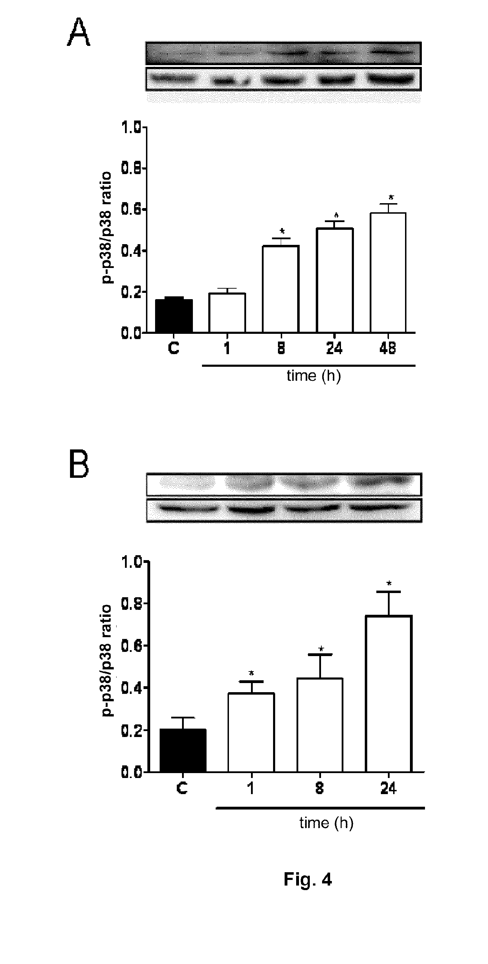

Figure 4 shows the effects of D-glucose at 50 mM (A) and 83 mM (B) on the phosphorylation of p38 mitogen-activated protein kinase (MAPK) in rat PMCs. Upper panel: a representative experiment. Lower panel: densitometric analysis of the normalized phospho-p38 levels with respect to the cells treated with the carrier (V). The data is expressed as mean ± SEM, n=3. * p <0.05 compared to the carrier (V).

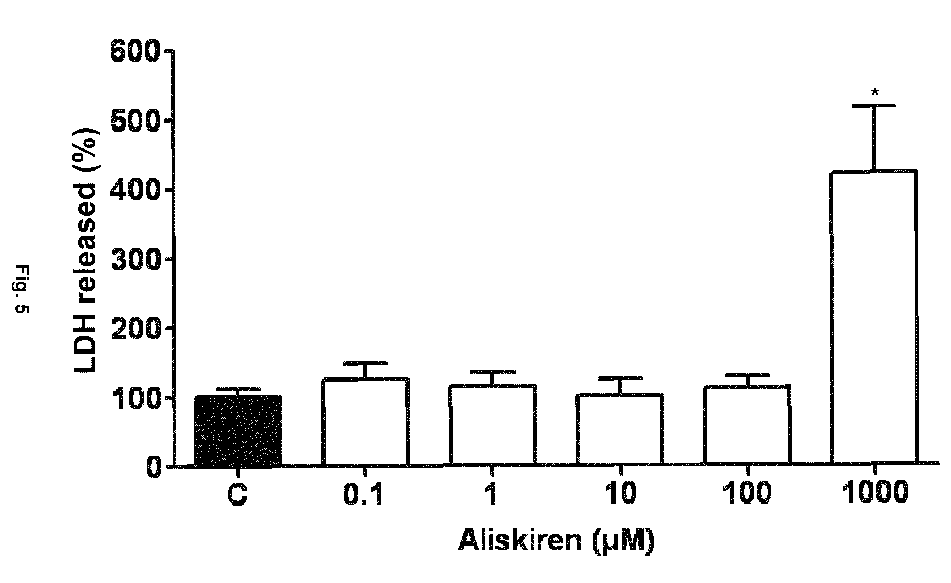

Figure 5 shows the toxic effects of aliskiren on rat PMCs. The cells were treated with carrier or aliskiren for 72 hours and cell viability was evaluated quantifying the LDH released into the culture medium. The data is expressed as mean ± SEM, n=12. * p <0.05 compared to the control (carrier).

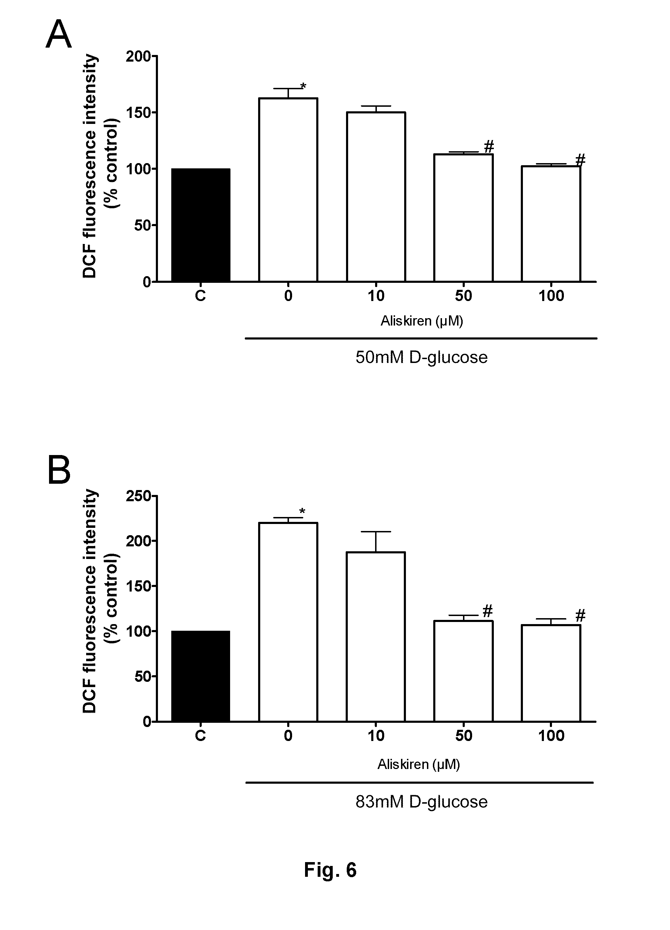

Figure 6 shows the effects of aliskiren on the changes produced by D-glucose at 50 mM (A) and 83 mM (B) in the production of reactive oxygen species (ROS) on rat PMCs. The cells were incubated in D-glucose at 50 mM (A) and 83 mM (B) for 8 hours in the presence or absence of aliskiren (10-100 µM) and dichlorofluorescein (DCF) intensity was measured using flow cytometry, as described in Material and Methods. The data is expressed as mean ± SEM, n=3. * p <0.05 compared to untreated cells (control). # p <0.05, compared to cells treated with D-glucose in the presence of carrier (0).

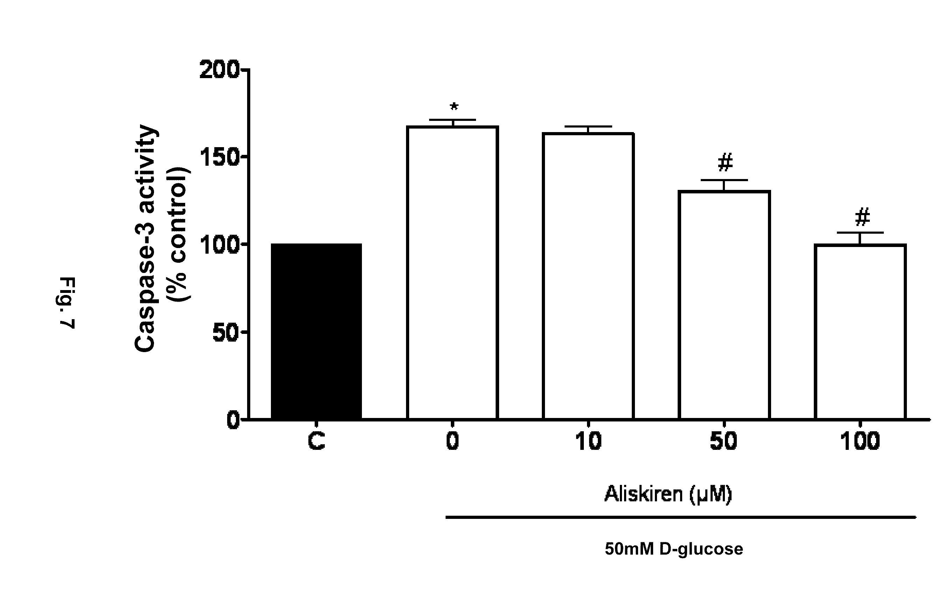

Figure 7 shows the effects of aliskiren on the changes produced by D-glucose at 50 mM in caspase-3 activity in rat PMCs. The cells were incubated in D-glucose at 50 mM for 24 hours in the presence or absence of aliskiren (10-100 µM), and caspase-3 activity was measured as described in Material and Methods. The data is expressed as mean ± SEM, n=3. * p <0.05 compared to the control (C). # p <0.05 compared to untreated cells (control). # p <0.05, compared to cells treated with D-glucose in the presence of carrier (0).

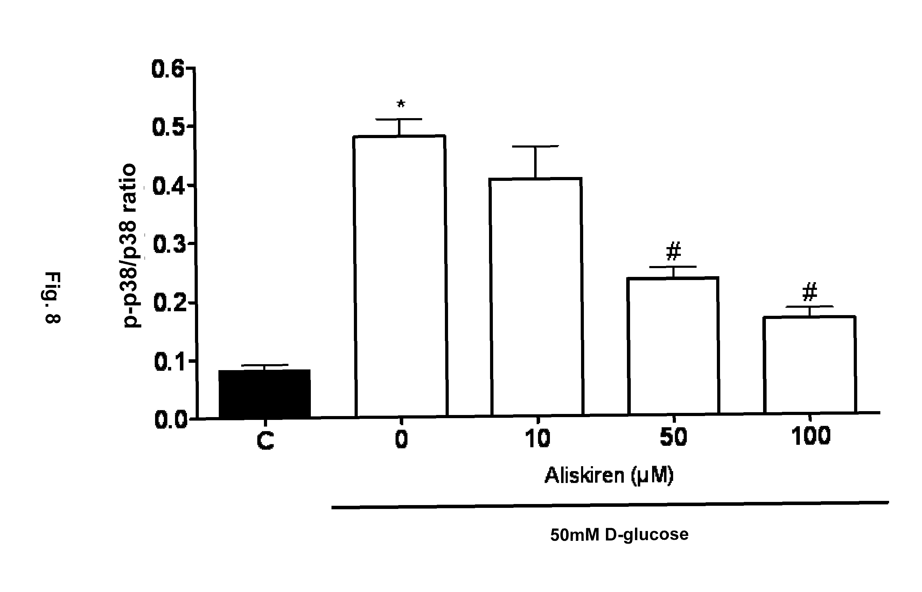

Figure 8 shows the effects of aliskiren on the changes produced by D-glucose at 50 mM in the phosphorylation of p38 mitogen-activated protein kinase (MAPK) in rat PMCs. Densitometric analysis of the phosphor-p38 levels normalized with respect to the controls (C). The data is expressed as mean ± SEM, n=3. * p <0.05 compared to untreated cells (control). # p <0.05, compared to cells treated with D-glucose in the presence of carrier (0).

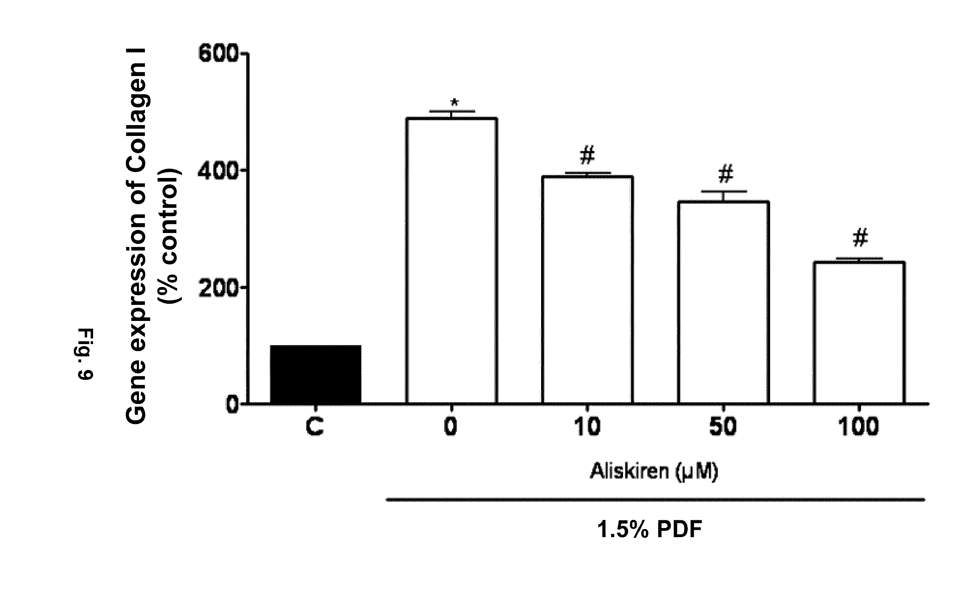

Figure 9 shows the effects of aliskiren on the gene expression of Collagen I in rat PMCs exposed to PDFs with glucose at 1.5% (PDF 1.5%) diluted 1:1 in culture medium. After incubating the rat PMCs in 1.5% PDF diluted 1:1 in culture medium for 24 hours, in the presence or absence of carrier or different concentrations of aliskiren (10-100 µM), total RNA was isolated and real-time RT-PCR was carried out as described in Material and Methods. The data is expressed as mean ± SEM, n=3. * p <0.05 compared to untreated cells (control). # p <0.05, compared to cells treated with 1.5% PDF in the presence of carrier (0).

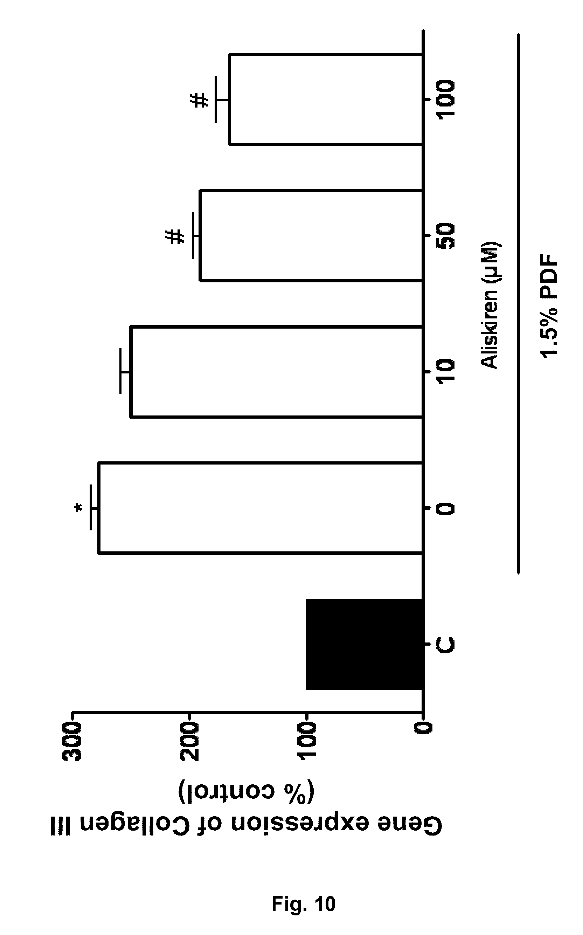

Figure 10 shows the effects of aliskiren on the gene expression of Collagen III in rat PMCs exposed to PDFs with glucose at 1.5% (PDF 1.5%) diluted 1:1 in culture medium. After incubating the rat PMCs in 1.5% PDF diluted 1:1 in culture medium for 24 hours, in the presence or absence of aliskiren (10-100 µM), total RNA was isolated and real-time RT-PCR was carried out as described in Material and Methods. The data is expressed as mean ± SEM, n=3. * p <0.05 compared to untreated cells (control). # p <0.05, compared to cells treated with 1.5% PDF in the presence of carrier (0).

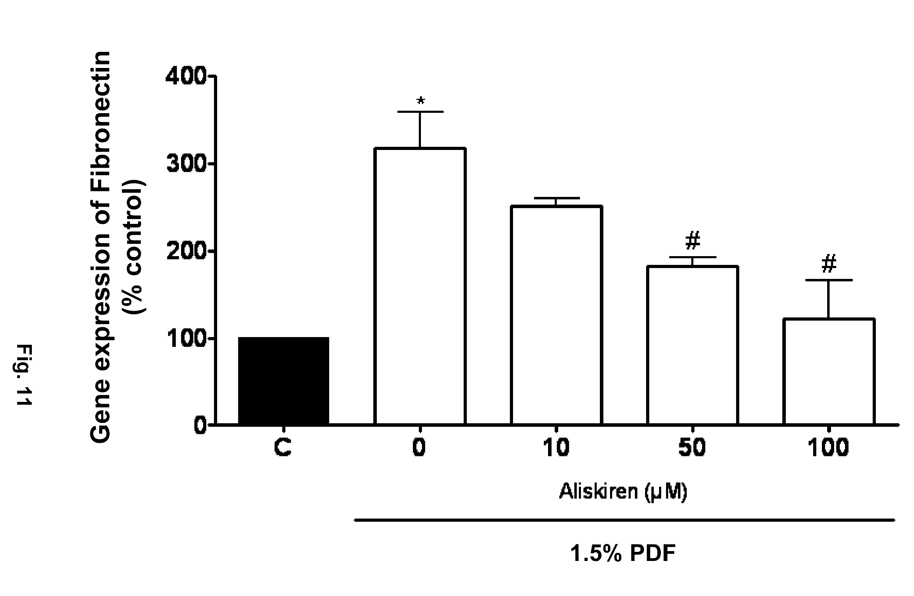

Figure 11 shows the effects of aliskiren on the gene expression of fibronectin in rat PMCs exposed to PDFs with glucose at 1.5% (PDF 1.5%) diluted 1:1 in culture medium. After incubating the rat PMCs in 1.5% PDF diluted 1:1 in culture medium for 24 hours, in the presence or absence of carrier or different concentrations of aliskiren (10-100 µM), total RNA was isolated and real-time RT-PCR was carried out as described in Material and Methods. The data is expressed as mean ± SEM, n=3. * p <0.05 compared to untreated cells (control). # p <0.05, compared to cells treated with 1.5% PDF in the presence of carrier (0).

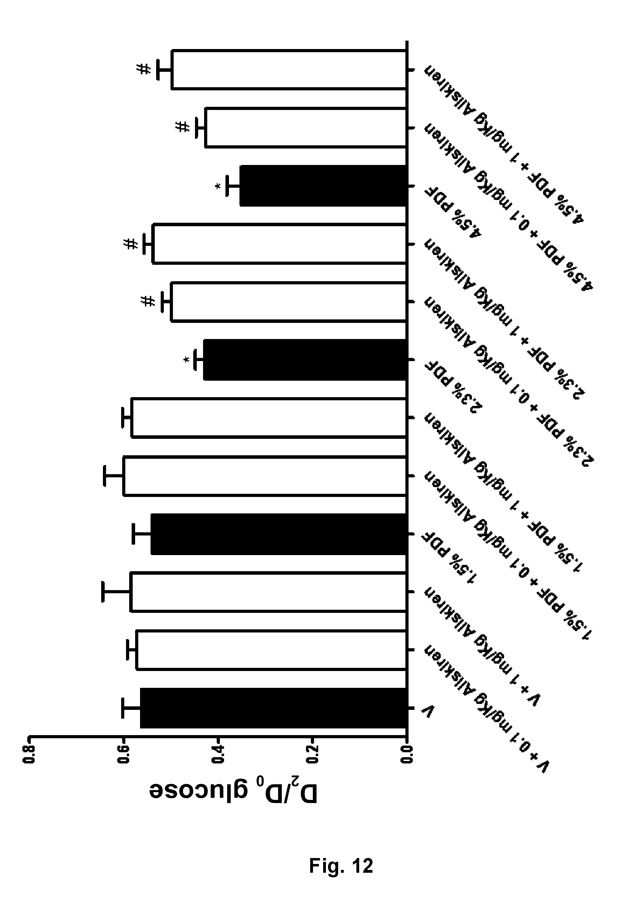

Figure 12 shows levels of D₂/D₀ glucose after dialysis over a period of four weeks to PDFs in rats. The rats were exposed daily for four weeks to carrier (saline serum), 1.5% PDF, 2.3% PDF or 4.5% PDF supplemented by carrier, 0.1 mg/Kg aliskiren or 1 mg/Kg aliskiren. The data are expressed as mean ± SEM, n=6 rats per group. * p <0.05, compared to the group treated with carrier in the absence of aliskiren (carrier). # p <0.05, compared to their respective groups treated with PDFs in the absence of aliskiren.

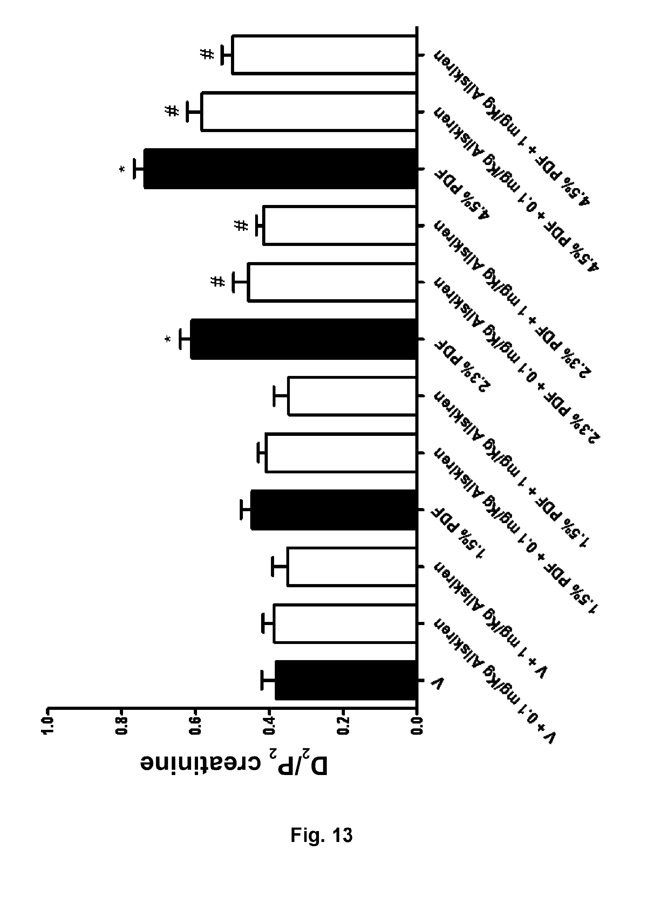

Figure 13 shows D₂/P₂ creatinine levels after dialysis over a period of four weeks to PDFs in rats. The rats were exposed daily for four weeks to carrier (saline serum), 1.5% PDF, 2.3% PDF or 4.5% PDF supplemented by carrier, 0.1 mg/Kg aliskiren or 1 mg/Kg aliskiren. The data is expressed as mean media ± SEM, n=6 rats per group. * p <0.05, compared to the group treated with carrier in the absence of aliskiren (carrier). # p <0.05, compared to their respective groups treated with PDFs in the absence of aliskiren.

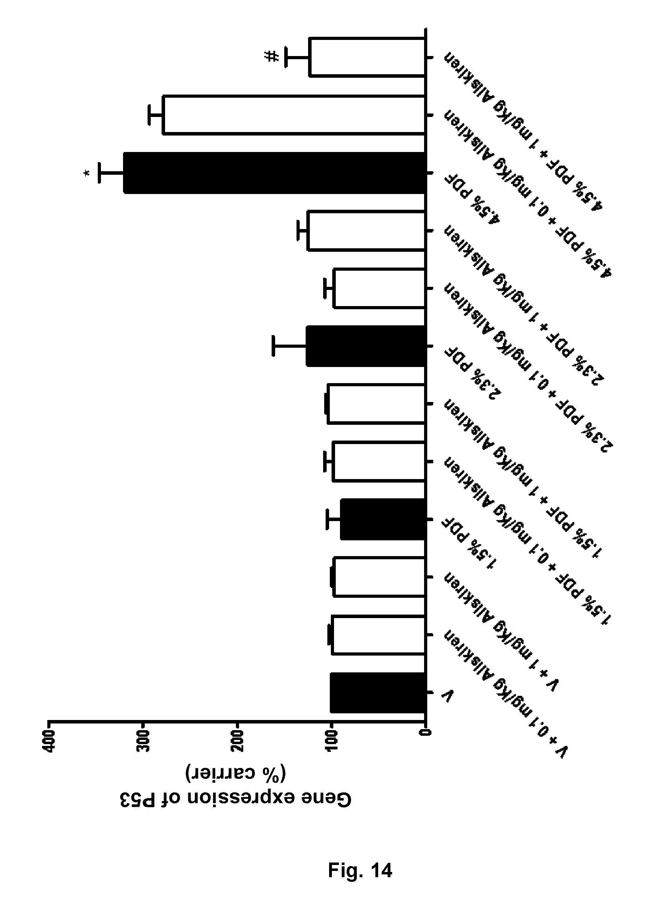

Figure 14 shows gene expression of p53 after dialysis over a period of four weeks to PDFs in rats. The rats were exposed daily for four weeks to carrier (saline serum), 1.5% PDF, 2.3% PDF or 4.5% PDF supplemented by, 0.1 mg/Kg aliskiren or 1 mg/Kg aliskiren. The data is expressed as mean ± SEM, n=6 rats per group. * p <0.05, compared to the group treated with carrier in the absence of aliskiren (carrier). # p <0.05, compared to their respective groups treated with PDFs in the absence of aliskiren.

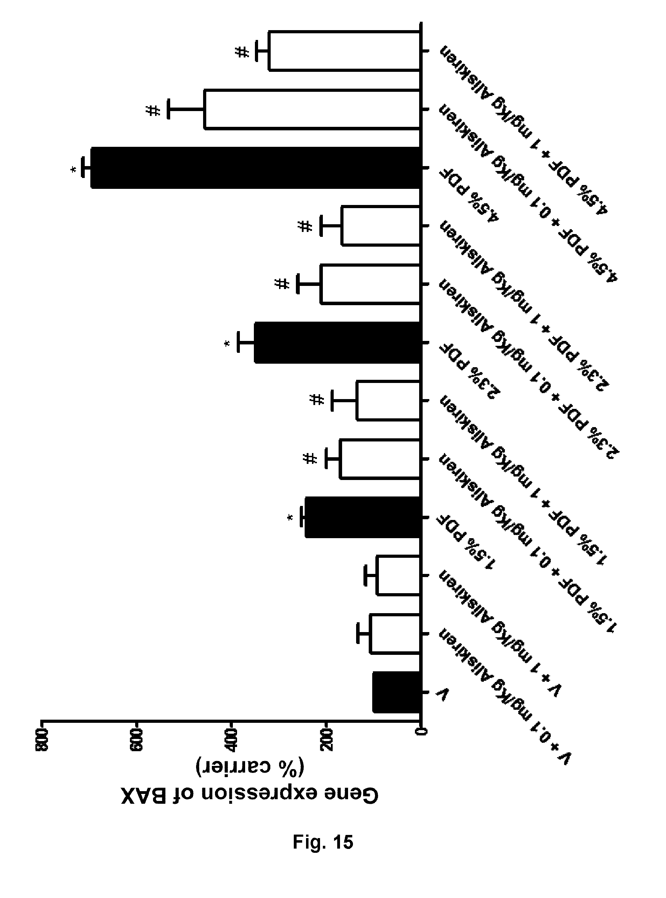

Figure 15 shows the levels of RNAm for BAX after dialysis over a period of four weeks to PDFs in rats. The rats were exposed daily for four weeks to carrier (saline serum), 1.5% PDF, 2.3% PDF or 4.5% PDF supplemented by carrier, 0.1 mg/Kg aliskiren or 1 mg/Kg aliskiren. The data is expressed as mean ± SEM, n=6 rats per group. * p <0.05, compared to the group treated with carrier in the absence of aliskiren (carrier). # p <0.05, compared to their respective groups treated with PDFs in the absence of aliskiren.

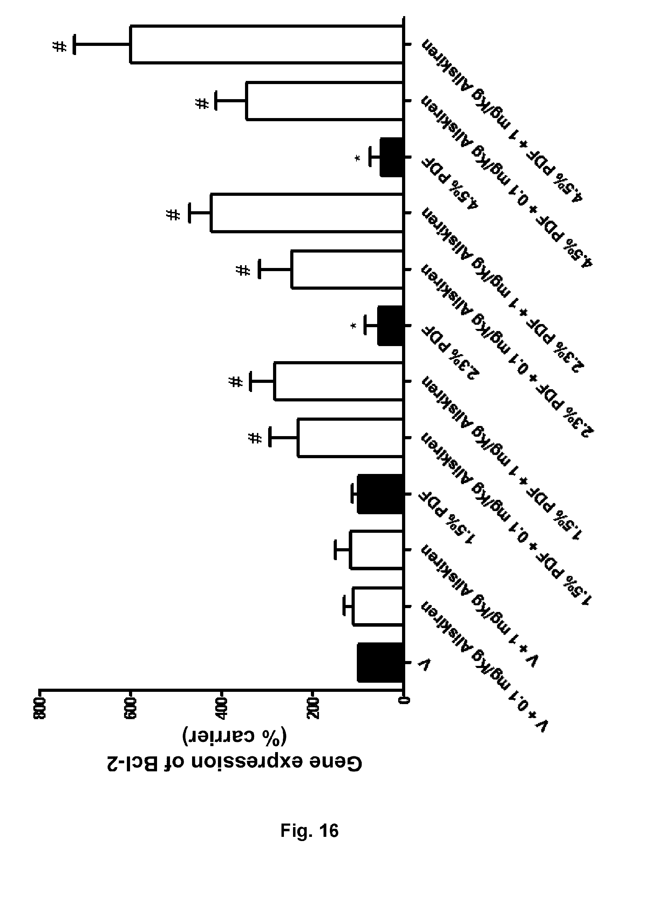

Figure 16 shows levels of RNAm for Bcl-2 after dialysis over a four week period to PDFs in rats. The rats were exposed daily for four weeks to carrier (saline serum), 1.5% PDF, 2.3% PDF or 4.5% PDF supplemented by carrier, 0.1 mg/Kg aliskiren or 1 mg/Kg aliskiren. The data is expressed as mean ± SEM, n=6 rats per group. * p <0.05, compared to the group treated with carrier in the absence of aliskiren (carrier). # p <0.05, compared to their respective groups treated with PDFs in the absence of aliskiren.

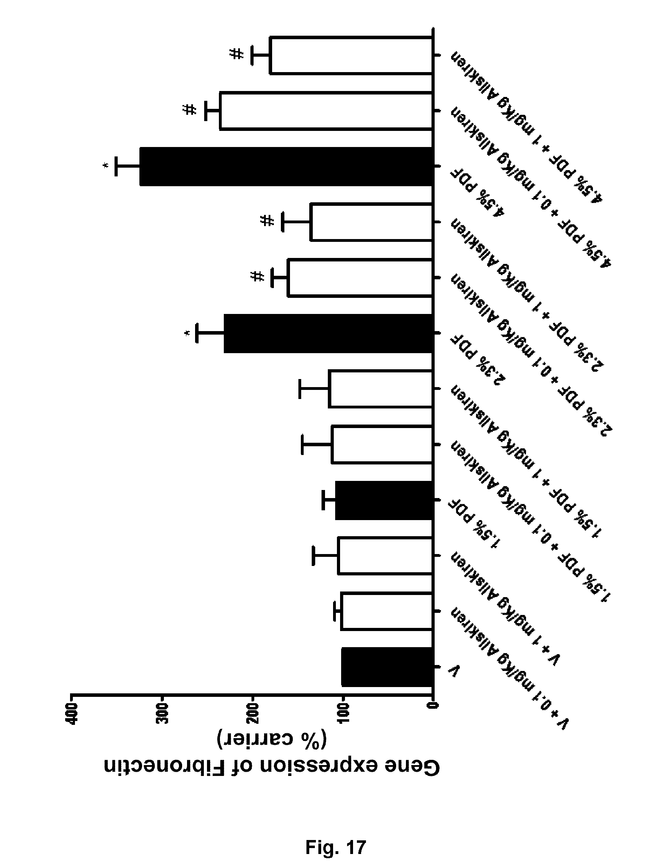

Figure 17 shows levels of RNAm for fibronectin after dialysis over a four week period to PDFs in rats. The rats were exposed daily for four weeks to carrier (saline serum), 1.5% PDF, 2.3% PDF or 4.5% PDF supplemented by carrier, 0.1 mg/Kg aliskiren or 1 mg/Kg aliskiren. The data is expressed as mean ± SEM, n=6 rats per group. * p <0.05, compared to the group treated with carrier in the absence of aliskiren (carrier). # p <0.05, compared to their respective groups treated with PDFs in the absence of aliskiren.

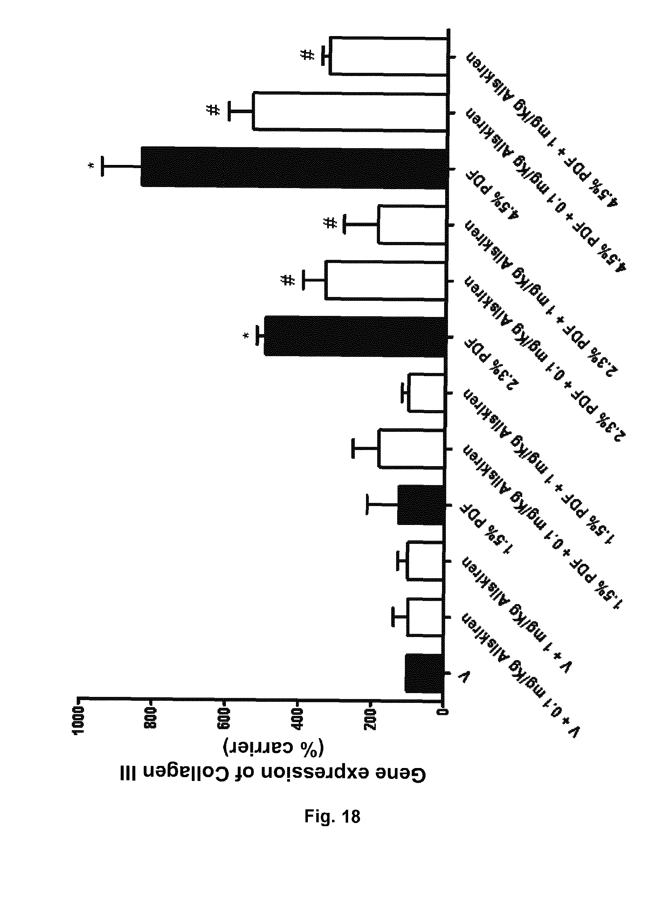

Figure 18 shows levels of RNAm for Collagen III after dialysis over a four week period to PDFs in rats. The rats were exposed daily for four weeks to carrier (saline serum), 1.5% PDF, 2.3% PDF or 4.5% PDF supplemented by carrier, 0.1 mg/Kg aliskiren or 1 mg/Kg aliskiren. The data is expressed as mean ± SEM, n=6 rats per group. * p <0.05, compared to the group treated with carrier in the absence of aliskiren (carrier). # p <0.05, compared to their respective groups treated with PDFs in the absence of aliskiren.

### EXAMPLES OF EMBODIMENT OF THE INVENTION

The following examples illustrate the present invention. However, these examples are non-limiting. They are merely informative and in no case limit the methodologies used, which can be altered for the purpose of achieving similar results.

In this specification, the symbols and conventions used in these processes, diagrams and examples consist of those used in the International System and contemporary scientific literature, for example, the Journal of Medicinal Chemistry. Unless indicated otherwise, all the materials were obtained from commercial suppliers and used without additional purification. Specifically, the following abbreviations can be used in the examples and throughout the specification: g (grams); mg (milligrams); Kg (kilograms); mL (millimetres); µL (microlitres); mmol (millimoles); M.P. (melting point); Hz (hertz); MHz (megahertz); δ (chemical displacement); ppm (parts per million); s (singlet); d (doublet); t (triplet); q (quartet); c (quintet); m (multiplet); J (coupling constant); NMR (nuclear magnetic resonance); MS (mass spectrum); ES (electrospray); m/z (mass/load ratio); Anal. (Elemental Analysis); Yld (Yield); TEA (triethylamine); CH₂Cl₂ (dichloromethane); CDCl₃ (deuterated chloroform); DMSO (dimethylsulphoxide); i.p. (parenteral administration). All temperatures are expressed in °C (degrees Celsius).

### Examples

### Rat peritoneal mesothelial cell cultures.

Rat peritoneal mesothelial cells were isolated by enzymatic digestion in accordance with protocols described in Hjelle JT, Golinska BT, Waters DC, et al. Perit Dial Int 1989; 9: 341-7*.* Briefly, female rats weighing 200-400 g of the *Spragle-Dawley* strain were sacrificed pursuant to Royal Decree 1201/2005, of 10 October, on protection of animals used for experimental and other scientific purposes. The abdominal cavity was quickly opened and the abdominal wall (peritoneum and smooth muscle) extracted under sterile conditions. The mesothelial cells were separated from the inner surface of the abdominal wall by enzymatic digestion, incubating said surface in 199 culture medium (Sigma) and 0.5 mg/ml of collagenase (Sigma) for 30 minutes at 37°C. After incubation, the digested abdominal wall surface was scraped to fully release the adhered mesothelial cells. The mesothelial cells obtained were seeded in culture dishes for growth and study thereof and were maintained in 199 medium supplemented with 10% foetal bovine serum, 100U/ml penicillin and 100µg/ml streptomycin. After each culture, the presence of peritoneal mesothelial cells was confirmed by its morphological appearance and the expression of specific markers. Cells were used between runs 3 and 6.

### In vivo studies

### Animals

Seventy-eight female rats of the Sprague-Dawley strain weighing 200-240 g (Charles River Breeding Laboratories) were used for the in vivo experiments. The animals were housed in an atmosphere at a constant temperature, with twelve-hour cycles of light and twelve hours of darkness. The feed and drink were administered ad libitum. All of the experimental protocols were carried out pursuant to European Directive "European Community Council Directive 86/609/EEC". Additionally, animal experiments were approved by the Animal Care and Use Committee of the University Hospital Complex of Albacete (Spain). Additionally, animal experiments are approved by the Animal Care and Use Committee of the Complejo Universitario Hospitalario de Albacete (Spain).

### Experimental design of in vivo studies and Peritoneal Equilibrium Test (PET)

For in vivo studies, 78 female rats of the Sprague-Dawley female strain weighing 200-240 g (Charles River Breeding Laboratories) were used: fifty-four rats were assigned to the group of animals which had been administered high-glucose peritoneal dialysis solutions (PDF), which were divided into three different subgroups with eighteen rats each which were administered, respectively, three PDFs having different glucose concentrations (***Fresenius*** Medical Car, Bad Homburg, Germany). Additionally, eighteen rats were administered a carrier (saline serum) and another six rats were used as a control group (not dialysed). The eighteen rats of the "carrier" group and those belonging to groups 1.5% PDF, 2.3% PDF and 4.5% PDF, were divided into another three subgroups of six rats each. The PDFs administered to each of these groups were complemented with saline serum, with 0.1 mg/Kg/day aliskiren (Novartis Pharmaceuticals, Cambridge, MA, USA) or with 1 mg/Kg/day aliskiren. For peritoneal dialysis, the rats received 20 ml of the corresponding PDF through a 22G needle, daily over a four week period.

At the end of the experimental period (four weeks), 30 ml of a commercial PDF with 2.3% glucose (***Fresenius*** Medical Car, Bad Homburg, Germany) was administered intraperitoneally using a 22G needle. Two hours later, the animals were anaesthetized intraperitoneally with pentobarbital (50 mg/kg) and blood was drawn by cardiac puncture. Next, a midline abdominal incision was made and the residual volume was collected from the peritoneum using a syringe. During the PET, the animals were awake and had free access to water and feed. Their body weight was measured at the start and end of the experimental period. No rat died and all the animals appeared to be in good health during the study, which includes repeated PDF injections.

The blood and dialysate samples were centrifuged and stored at -20°C. The concentrations of creatinine and glucose in the serum and dialysate were measured using enzymatic methods. The peritoneal transport of solutes was calculated, on one hand, as the glucose concentration in the dialysate 2 hours after the PET relative to the initial glucose concentration in the PDF (D₂/D₀ glucose) and, on the other, as the dialysate/plasma creatinine concentration ratio (D₂/P₂ creatinine) 2 h after the PET. At the end of the PET, the animals were sacrificed using an overdose of anaesthetic and the peritoneal mesothelial cells (PMCs) isolated and cultured as described later in this specification.

### Preparation of the peritoneal dialysis solutions of the present invention

The peritoneal dialysis solutions were prepared by adding the quantities of aliskiren described in Table 2 and the quantities of glucose described in Table 3 to medium M199, the composition of which is shown in Table 1. The combinations of each of the aliskiren concentrations that appear in Table 2 and the glucose concentrations that appear in Table 3 were studied.

**TABLE 1. Composition of 199 medium.**

| Inorganic salts (electrolytes) | g/L | pH buffer tampon | g/L | Osmotic agent | g/L |
|---|---|---|---|---|---|
| CaCl2 + 2H2O | 0.2 | NaHC03 | 2.2 | Glucose | 1 |
| Fe(N03)3 + | 0.00 | | | | |
| 9H2O | 072 | | | | |
| MgS04 | 0.09 | | | | |
| KCI | 767 | | | | |
| Sodium | 0.4 | | | | |
| acetate | 0.05 | | | | |
| NaCl | 6.8 | | | | |
| NaH2PO4 | 0.12 | | | | |
| | 2 | | | | |

| Amino acids | g/L | Vitamins | g/L | Other | g/L |
|---|---|---|---|---|---|
| L-Alanine | 0.02 | Ascorbic acid Na | 5.66E | Adenine sulphate | 0.01 |
| L-Arginine HCl | 5 | D-Biotin | -05 | Adenosine | 0.001 |
| L-Aspartic acid | 0.07 | Calcipherol | 0.000 | triphosphate 2Na | 0.000 |
| L-Cystine HCl | 0.03 | Choline Cl | 01 | Adenosine | 239 |
| + H2O | 0.00 | Folic acid | 0.000 | monophosphate | 0.000 |
| L-Cysteine | 011 | Menadione (sodium | 1 | Na | 2 |
| 2HCl | 0.02 | bisulphate) | 0.000 | Cholesterol | 0.000 |
| L-Glutamic | 6 | Myo-inositol | 5 | Deoxyribose | 5 |
| acid | 0.06 | Niacinamide | 0.000 | Glutation | 0.000 |
| L-Glutamine | 68 | Nicotinic acid | 01 | (reduced) | 05 |
| Glycine | 0.1 | P-Amino benzoic | 0.000 | Guanine HCl | 0.000 |
| L-Histidine HCl | 0.05 | acid | 016 | Hypoxanthine | 3 |
| + H2O | 0.02 | D-Pantothenic acid | 0.000 | Phenol red Na | 0.000 |
| Hydroxy-L- | 188 | ½Ca | 05 | TWEEN 80 | 3 |
| Proline | 0.01 | Pyridoxal HCl | 0.000 | Ribose | 0.021 |
| L-Isoleucine | 0.02 | Pyridoxine HCl | 025 | Thymine | 3 |
| L-Leucine | 0.06 | Retinol acetate | 0.000 | Uracil | 0.02 |
| L-Lysine HCl | 0.07 | Riboflavin | 025 | Xanthine Na | 0.000 |
| L-Methionine | 0.01 | DL-alpha- | 0.000 | | 5 |
| L- | 5 | Tocopherol | 05 | | 0.000 |
| Phenylalanine | 0.02 | phosphate Na | 0.000 | | 3 |
| L-Proline | 5 | Thiamine HCl | 01 | | 0.000 |
| L-Serine | 0.04 | | 0.000 | | 3 |
| L-Threonine | 0.02 | | 025 | | 0.000 |
| L-Tryptophan | 5 | | 0.000 | | 344 |
| L-Tyrosine 2N | 0.03 | | 025 | | |
| at 2H2O | 0.01 | | 0.000 | | |
| L-Valine | 0.05 | | 014 | | |
| | 766 | | 0.000 | | |
| | 0.02 | | 01 | | |
| | 5 | | 0.000 | | |
| | | | 01 | | |
| | | | 0.000 | | |
| | | | 01 | | |

**TABLE 2. Aliskiren concentrations of the peritoneal dialysis solutions.**

| Aliskiren concentration (µM) | Quantity of aliskiren added (g/L) |
|---|---|
| 0.1 | 0.000055 |
| 1 | 0.00055 |
| 10 | 0.0055 |
| 50 | 0.0275 |
| 100 | 0.055 |
| 1000 | 0.55 |

**TABLE 3. Glucose concentrations of the peritoneal dialysis solutions.**

| Glucose concentration (mM) | Quantity of glucose added (g/L) |
|---|---|
| 5.6 | 0 |
| 50 | 8 |
| 83 | 14 |

### Cytotoxicity studies

Toxicity tests were conducted on the rat peritoneal mesothelial cell cultures, determining the activity of the lactate dehydrogenase (LDH) enzyme (Posadas I, López-Hernández B, Clemente MI, et al. Pharm Res 2009; 26: 1181-91). These studies were carried out to study the toxicity of the peritoneal dialysis solutions of the present invention.

To this end, the cells were seeded on 24-well plates and exposed to the peritoneal dialysis solutions object of the present invention to study their toxicity. The toxic effects were evaluated measuring cell membrane rupture and the consequent release of LDH to the supernatant through the CytoTox96® kit (Promega). The cells were mechanically removed, washed with PBS and centrifuged at 10,000 rpm for 10 minutes. Absorbance of the lysate and cell supernatant was obtained using a microplate spectrophotometer at a wavelength of 490 nm, following the manufacturer's instructions.

### Evaluation of changes in the concentration of reactive oxygen species using flow cytometry

After washing with PBS, the cells were incubated with 5.6-chloromethyl-2.7-dichlorodihydrofluorescein diacetate (CM-H2DCFDA, Invitrogen) for 15 minutes at 37°C, following the standard protocol (Lee HB, 2004). After incubation, the conditioned media were collected and the cells were washed with PBS and trypsinized. The total cells -living and dead- present in the suspension resulting from binding the cell trypsinate to the concentrated medium were centrifuged at 3000 x g for 5 minutes at 4°C. Finally, the cells were washed with PBS and re-suspended in culture medium 199 for analysis thereof in the flow cytometer (FACSCalibur, Becton-Dickinson, Franklin Lakes, NJ, USA). The production of reactive oxygen species was calculated on the basis of evaluation of 10,000 cells per experimental condition.

### Evaluation of the phosphorylation/dephosphorylation of p38 MAPK by Western blot

In order to obtain the total protein of the cell cultures, the culture medium was collected and replaced with PBS. The cells were removed by means of trypsinisation and bound to the collected medium. The protein-enriched pellet resulting from centrifuging at 3000 x g for 5 minutes at 4°C was re-suspended in a lysate buffer (100mM HEPES, 5mM DTT, 5mM EGTA, 0.04% Nonidet P-40 and 20% glycerol) with protease inhibitors and phosphatases and incubated for 60 minutes at 4°C. After incubation, the samples were centrifuged at 13,000 rpm for 15 minutes at 4°C and the supernatants preserved at -80°C. The protein content of the cell lysates was determined using the Bradford method, following the standard protocol (Vorobiov M, Malki M, Shnaider A, et al. Perit Dial Int 2008; 28: 648-54). A quantity of 20 µg of protein/well of each sample was loaded onto 10% polyacrylamide gels. The gels were transferred by electrophoresis to nitrocellulose membranes using a semi-dry blotter. The proteins bound to nitrocellulose were viewed by Ponceau S staining and then blocked with TTBS (50 mM Tris, pH 7.5, 200 mM NaCl, 0.1 % Tween) with 5% of skimmed milk and, subsequently, incubated in the p38 MAPK or phospho-p38 MAPK primary antibody (Cell Signalling Technology) all night at 4°C. After washing in TTBS, the secondary antibody was applied for 1 hour at ambient temperature. Detection was carried out by chemiluminescence (ECL). Band intensity was quantified by grey levels using an appropriate image analysis system (Quantity One).

### Determination of caspase-3 activity by ELISA

In order to determine caspase-3 activity (Posadas I, Vellecco V, Santos P, et al. Br J Pharmacol 2007; 150: 577-85*),* the rat mesothelial cells were seeded on 6-well plates until reaching a confluence of 80%. After exposure to the peritoneal dialysis solutions with aliskiren, the culture medium was collected and replaced with PBS. The cells were removed by trypsinisation and bound to the collected medium. The protein-enriched pellet resulting from centrifuging at 3000 x g for 5 minutes at 4°C was re-suspended in a lysate buffer (100mM HEPES, 5mM DTT, 5mM EGTA, 0.04% Nonidet P-40 and 20% glycerol) and incubated for 60 minutes at 4°C. After incubation, the samples were centrifuged at 13,000 rpm for 15 minutes at 4°C and the protein concentration in the supernatants was determined using the Bradford method, following the standard protocol. The cell extracts (40 µg of protein) were incubated in a reaction buffer (25mM HEPES, 10% sucrose, 0.1% 3-[(3-cholamide propyl)-dimethylammonio]-2-hydroxy-1-propanesulfonic acid, 10mM DTT) that contained the fluorescent substrate Asp-Glu-Val-Asp-7-amino-4 trifluoromethyl-coumaryl (Z-DEVD-AFC) 50µM, at 37°C for 1 hour. Fluorescence emission was determined on a spectrofluorimeter (Tecan, Austria) at an excitation wavelength of 400 nm and an emission wavelength of 505 nm.

### Real-time RT-PCR

The RNA expression was evaluated by real-time RT-PCR in PMCs. The total RNA was isolated using a commercial reactive (Tripure, Sigma, St. Louis, MO), following the manufacturer's instructions. RNA quality and concentration was evaluated by spectrophotometry (Infinite 200, Tecan, Salzburg, Austria) using 1 µl of the RNA sample. Total RNA was always tested in an agarose gel in order to confirm the integrity of the18S and 28S mRNA bands. cDNA was synthesised using the High Capacity cDNA Reverse Transcription Kit (Applied Biosystems, Foster City, CA). In the case of real-time RT-PCR, cDNA was amplified using SYBR Green PCR Master mix with StepOne Real-Time PCR System and software StepOne v2.0 (Applied Biosystems, Foster City, CA). The following primer pairs were used for amplification: fibronectin, 5'-GCA-CAG-GGG-AAG-AAA-AGG-AG-3' (sense) y 5'-TTG-AGT-GGA-TGG-GAG-GAG-AG-3' (antisense); Collagen I, 5'-TCA-CCT-ACA-GCA-CGC-TTG-3' (sense) and 5'-GGT-CTG-TTT-CCA-GGG-TTG-3'(antisense); Collagen III, 5'-ATA-TCA-AAC-ACG-CAA-GGC-3' (sense) and 5'-GAT-TAA-AGC-AAG-AGG-AAC-AC-3' (antisense); P53, 5'-CCT-CCT-CAG-CAT-CTT-ATC-CG-3' (sense) and 5'-CAC-AAA-CAC-GCA-CCT-CAA-A-3' (antisense); BAX, 5'-GAT-GCG-TCC-ACC-AAG-AA-3' (sense) and 5'-AGT-AGA-AGA-GGG-CAA-CCA-C-3' (antisense); and Bcl-2, 5'-CCC-AAG-GGA-AGA-CGA-TG-3' (sense) and 5'-GAG-CGG-GTA-GGG-AAA-GA-3'(antisense). The primer sequences have an annealing temperature similar to 60^{a}C. The real-time RT-PCR reaction was carried out at 95°C for 10 minutes, followed by 40 cycles of: 95°C for 15 seconds and 60°C for 1 minute. The dissociation curves were analysed to confirm the amplification of a single PCR product. All the samples were processed in triplicate. In each experiment, the mean cycle threshold (CT) was calculated from triplicates for each of the genes studied, enabling comparison of the gene expression after different treatments (Methods 25, 402-408 (2001). In order to normalize the data, the gene expression of β-actin was used as endogenous control.

### Data collection and analysis

Each value obtained will correspond to a minimum of three experiments conducted in duplicate. All the data is presented as mean ± SEM. The differences between the groups, for each of the parameters analysed, were analysed using a nonparametric ANOVA test (Kruskal-Wallis), followed by an ad hoc test (Dunnett). A p value less than or equal to 0.05 was considered significant. SPSS 13.0 (Chicago, IL) software was used to carry out the statistical analysis.

### Results

The mesothelial cell culture used in the experiments of this invention shows HBME-1 reactivity (Figure 1).

Increasing exposure time to different glucose concentrations in the dialysis liquid is toxic to the mesothelial cells, constituting the basis of toxicity of the peritoneal dialysis solutions with respect to the peritoneum. It can be observed that an increase in exposure time to glucose increases the production of free radicals in the mesothelial cells (Figure 2).

Due to this production of free radicals, effector mechanisms of cell death, such as caspase-3 activation, are activated (Figure 3).

Exposure to solutions with high glucose concentrations increases phosphorylation of p38 protein, which is considered a toxicity index in mesothelial cells (Figure 4).

The addition of aliskiren to the culture medium did not show toxicity with respect to mesothelial cells until reaching a concentration of 1 mM (Figure 5).

The addition of aliskiren to the dialysis medium prevented both production of reactive oxygen species (Figure 6) and activation of caspase (Figure 7) and phosphorylation of protein p38 (Figure 8) in cultured peritoneal cells.

The addition of aliskiren to the dialysis medium lowered the RNAm levels for markers of peritoneal fibrosis, collagen I (Figure 9), collagen III (Figure 10) and fibronectin (Figure 11), in cultured peritoneal cells.

The presence of aliskiren in the dialysis medium reduced peritoneal damage in vivo in dialysed rats (Figures 12 and 13).

The presence of aliskiren during chronic peritoneal analysis in rats reduced RNAm expression in peritoneal cells obtained from said rats, for pro-apoptotic proteins p53 (Figure 14) and Bax (Figure 15).

The presence of aliskiren during chronic peritoneal dialysis in rats increased RNAm expression in peritoneal cells obtained from said rats for the anti-apoptotic protein Bcl-2 (Figure 16).

The presence of aliskiren during chronic peritoneal dialysis in rats increased RNAm expression in peritoneal cells obtained from said rats for markers of peritoneal fibrosis, fibronectin (Figure 17) and collagen III (Figure 18).

## Claims

1. A peritoneal dialysis solution that comprises the following elements:
a) at least one electrolyte;
b) at least one buffer solution;
c) at least one osmotic pressure regulating agent; and
d) aliskiren or any of its derivatives.

2. A peritoneal dialysis solution, according to claim 1, wherein the electrolyte(s) are selected from the following group formed by: Na⁺, Mg²⁺, Ca²⁺, Cl⁻ or any combination thereof.

3. A peritoneal dialysis solution, according to claim 2, wherein each electrolyte has the following concentration range: Na 100-200 mmol/l; K 0-4 mmol/l; Mg 0.1-2 mmol/l; Ca 0.5-3 mmol/l; Cl 50-200 mmol/l.

4. A peritoneal dialysis solution, according to any of claims 1 to 3, wherein the buffer solution is selected from lactate, bicarbonate or a combination thereof.

5. A peritoneal dialysis solution, according to claim 4, wherein the concentration of the buffer solution ranges from 10 to 150 mmol/l.

6. A peritoneal dialysis solution, according to any of claims 1 to 5, wherein the osmotic agent is selected from the group formed by glucose, polyglucose, mannitol, glycerol, amino acids, polypeptides or any combination thereof.

7. A peritoneal dialysis solution, according to claim 6, wherein the quantity of osmotic agent to be added ranges from 100 to 600 mOsm/kg.

8. A peritoneal dialysis solution, according to any of claims 1 to 7, wherein the concentration of aliskiren or any of its derivatives ranges from 50nM to 1mM.

9. Use of the peritoneal dialysis solution for preparing a medicament.

10. Use of the dialysis solution for preparing a medicament for preventing and/or treating renal diseases.

11. Use of the dialysis solution for preparing a medicament for preventing and/or treating toxicity with respect to peritoneal mesothelial cells.

12. Use of the dialysis solution for preparing a medicament for preventing and/or reducing reactive oxygen species, phosphorylation of protein p39 MAPK and activation of caspase- 3.

13. Use of the dialysis solution for preparing a medicament for preventing and/or treating oxidative stress, apoptotic processes, expression of genes harmful to peritoneal cells and peritoneal damage caused by dialysis liquids.

14. Use of the dialysis solution for preparing a medicament for preventing and/or treating chronic renal failure.


SEQUENCE LISTING

<110>  Universidad de Castilla la Mancha

<120>  "Peritoneal dialysis solution"

<130>  PCT1874.7BIS

<140>  PCT/ES2011/070142
<141>  2011-03-04

<150>  P201030326
<151>  2010-03-05

<160>  13

<170>  PatentIn version 3.5

<210>  1
<211>  4
<212>  PRT
<213>  Artificial Sequence

<220>
<223>  Sequence binded to a fluorescent substrate

<400>  1

Asp Glu Val Asp
1


<210>  2
<211>  20
<212>  DNA
<213>  Artificial Sequence

<220>
<223>  Fibronectin sense primer

<400>  2
gcacagggga agaaaaggag                                                   20


<210>  3
<211>  20
<212>  DNA
<213>  Artificial Sequence

<220>
<223>  Fibronectin antisense primer

<400>  3
ttgagtggat gggaggagag                                                   20


<210>  4
<211>  18
<212>  DNA
<213>  Artificial Sequence

<220>
<223>  Collagen I sense primer

<400>  4

tcacctacag cacgcttg                                                    18


<210>  5
<211>  18
<212>  DNA
<213>  Artificial Sequence

<220>
<223>  Collagen I antisense primer

<400>  5
ggtctgtttc cagggttg                                                    18


<210>  6
<211>  18
<212>  DNA
<213>  Artificial Sequence

<220>
<223>  Collagen III sense primer

<400>  6
atatcaaaca cgcaaggc                                                    18


<210>  7
<211>  20
<212>  DNA
<213>  Artificial Sequence

<220>
<223>  Collagen III antisense primer

<400>  7
gattaaagca agaggaacac                                                  20


<210>  8
<211>  20
<212>  DNA
<213>  Artificial Sequence

<220>
<223>  P53 sense primer

<400>  8
cctcctcagc atcttatccg                                                  20


<210>  9
<211>  19
<212>  DNA
<213>  Artificial Sequence

<220>
<223>  P53 antisense primer

<400>  9
cacaaacacg cacctcaaa                                                   19


<210>  10
<211>  17

<212>  DNA
<213>  Artificial Sequence

<220>
<223>  BAX sense primer

<400>  10
gatgcgtcca ccaagaa                                                      17


<210>  11
<211>  19
<212>  DNA
<213>  Artificial Sequence

<220>
<223>  BAX antisense primer

<400>  11
agtagaagag ggcaaccac                                                    19


<210>  12
<211>  17
<212>  DNA
<213>  Artificial Sequence

<220>
<223>  Bcl- 2 sense primer

<400>  12
cccaagggaa gacgatg                                                      17


<210>  13
<211>  17
<212>  DNA
<213>  Artificial Sequence

<220>
<223>  Bcl- 2 antisense primer

<400>  13
gagcgggtag ggaaaga                                                      17